# EUROPEAN PATENT APPLICATION

(11) **EP 4 644 061 A1**
(43) Date of publication of application: **05.11.2025**
(21) Application number: 25151962.5
(22) Date of filing: 15.01.2025
(51) Int. Cl.: B25J 13/08, B62D 57/032

(54) **FOOT SKELETON, FOOT STRUCTURE, ROBOTIC LEG AND LEGGED ROBOT**

(30) Priority: 30.04.2024 CN 202410544563
(71) Applicant: Beijing Xiaomi Robot Technology Co., Ltd., Beijing 100176 (CN)
(72) Inventor: ZHU, Xiaolong, Beijing, 100176 (CN); LI, Yuanyuan, Beijing, 100176 (CN); WANG, Xirui, Beijing, 100176 (CN); CHEN, Yao, Beijing, 100176 (CN)
(74) Representative: Stöckeler, Ferdinand

(57) **Abstract**

A foot skeleton (20), a foot structure, a robotic leg, and a legged robot are provided. The foot skeleton (20) includes at least one deformation arm (50, 52), the at least one deformation arm (50, 52) is arranged along at least one of a length direction and a width direction of the foot skeleton (20), and a side portion of the at least one deformation arm (50, 52) includes a deformation hole (60). The at least one deformation arm (50, 52) is provided with at least one force detection unit (30).

## Description

### FIELD

The present invention relates to the field of robots, and more particularly to a foot skeleton, a foot structure, a robotic leg and a legged robot.

### BACKGROUND

A legged robot detects a motion state and a force condition of a foot to provide necessary reference information for a subsequent motion control. The usual legged robot is often installed with a force sensor at the robot foot, but generally with problems such as a complex structure, a low space utilization, a big volume, a large weight, a high cost, and a low integration.

### SUMMARY

The present invention provides a foot skeleton, a foot structure, a robotic leg and a legged robot, so as to solve at least a part of the problems in the related art.

In a first aspect, embodiments of the present invention provide a foot skeleton, the foot skeleton includes at least one deformation arm, the at least one deformation arm is arranged along at least one of a length direction and a width direction of the foot skeleton, a side portion of the at least one deformation arm includes a deformation hole, and the at least one deformation arm is provided with at least one force detection unit.

Optionally, the foot skeleton includes a plurality of grooves, the plurality of grooves are arranged along at least one of the length direction and the width direction of the foot skeleton, and the foot skeleton is separated by the plurality of grooves to form a plurality of deformation arms.

Optionally, each of two ends of the foot skeleton along the length direction includes a first groove, and each of the two ends of the foot skeleton along the length direction is separated by the first groove to form a plurality of first deformation arms; and/or each of two ends of the foot skeleton along the width direction includes a second groove, and each of the two ends of the foot skeleton along the width direction is separated by the second groove to form a plurality of second deformation arms.

Optionally, the first groove is in a shape of a slit, and each of the two ends of the foot skeleton along the length direction includes two first grooves, the two first grooves located at the same side of the foot skeleton are spaced apart along the width direction of the foot skeleton, and each of the two ends of the foot skeleton along the length direction is separated by the two first grooves located at the same side to form two first deformation arms and a main body portion located between the two first deformation arms.

Optionally, the two first grooves located at the same side along the length direction of the foot skeleton are symmetrically arranged with respect to a center line along the length direction of the foot skeleton, and the two first grooves located at the same side along the width direction of the foot skeleton are symmetrically arranged with respect to a center line along the width direction of the foot skeleton; and the two first deformation arms located at the same side along the length direction of the foot skeleton are symmetrically arranged with respect to a center line along the length direction of the foot skeleton, and the two first deformation arms located at the same side along the width direction of the foot skeleton are symmetrically arranged with respect to a center line along the width direction of the foot skeleton.

Optionally, the first groove has a rectangular shape, each of the two ends of the foot skeleton along the length direction includes one first groove, and each of the two ends of the foot skeleton along the length direction is separated by the first groove to form two first deformation arms.

Optionally, the foot skeleton further includes a weight reduction groove at a position adjacent to the first groove, the weight reduction groove has a rectangular shape and is in communication with the first groove, and a length of the weight reduction groove is less than a length of the first groove along the width direction of the foot skeleton.

Optionally, each of the two ends of the foot skeleton along the width direction includes a notch portion and two second grooves in communication with the notch portion, the two second grooves located at the same side of the foot skeleton along the width direction are spaced apart along the length direction of the foot skeleton, and each of the two ends of the foot skeleton along the width direction is separated by the two second grooves located at the same side to form two second deformation arms.

Optionally, an axial direction of the deformation hole extends along a width direction of the at least one deformation arm.

Optionally, a section of the deformation hole in a direction perpendicular to the axial direction of the deformation hole is in a shape of a ring.

Optionally, the deformation hole is in a shape of a runway circle.

Optionally, the deformation hole includes an opening end, and the opening end runs through a top surface or a bottom surface of the at least one deformation arm.

Optionally, the deformation hole includes a first hole portion and a second hole portion in communication with the first hole portion, the second hole portion includes the opening end, an axial direction of the first hole portion and an axial direction of the second hole portion both extend along the width direction of the at least one deformation arm, and the first hole portion and the second hole portion are connected by a bent portion.

Optionally, a diameter of the first hole portion and a diameter of the second hole portion are equal or a diameter of the first hole portion exceeds a diameter of the second hole portion; and/or the first hole portion and the second hole portion are perpendicular to each other; and/or the bent portion between the first hole portion and the second hole portion is an circular arc transition structure.

Optionally, each of two ends of the deformation hole along the axial direction runs through sidewalls at two sides of the at least one deformation arm along the width direction; or at least one of the two ends of the deformation hole along the axial direction runs through a sidewall of the at least one deformation arm along the width direction; or neither of the two ends of the deformation hole along the axial direction runs through sidewalls at two sides of the at least one deformation arm along the width direction.

Optionally, the force detection unit is arranged at a maximum strain position of the at least one deformation arm; and/or the force detection unit includes a stress patch.

In a second aspect, embodiments of the present invention provide a foot structure, the foot structure includes a force equalizing plate and at least one foot skeleton as described in the first aspect, and the at least one foot skeleton is connected to the force equalizing plate.

Optionally, the foot structure further includes a plurality of fasteners, the fastener passes through the deformation arm and is detachably connected to the force equalizing plate, portions of the foot skeleton and the force equalizing plate corresponding to the fastener abut against each other, and a gap is defined between remaining portions of the foot skeleton and the force equalizing plate; or the force equalizing plate and the foot skeleton are integrally formed, portions of the foot skeleton and the force equalizing plate corresponding to the force detection unit abut against each other, and a gap is defined between remaining portions of the foot skeleton and the force equalizing plate.

Optionally, the force equalizing plate is an integrally formed structure, the force equalizing plate includes a fore sole portion corresponding to a front end of the foot skeleton, a rear sole portion corresponding to a rear end of the foot skeleton, and a foot arch connected between the fore sole portion and the rear sole portion, and the fore sole portion, the rear sole portion and the foot arch are integrally formed; or the force equalizing plate includes a fore sole portion and a rear sole portion separated from each other, the fore sole portion corresponds to a front end of the foot skeleton and is connected to the foot skeleton, and the rear sole portion corresponds to a rear end of the foot skeleton and is connected to the foot skeleton.

Optionally, a middle of the foot arch includes a hollow-out portion, and a reinforce rib is arranged in the hollow-out portion; and/or a region of the foot skeleton corresponding to the fore sole portion is provided with a plurality of deformation arms, which are spaced apart from each other, along a circumferential direction of the fore sole portion; and/or a region of the foot skeleton corresponding to the rear sole portion is provided with a plurality of deformation arms, which are spaced apart from each other, along a circumferential direction of the rear sole portion.

Optionally, the foot structure further includes a flexible pad, and the flexible pad is arranged at a side of the force equalizing plate facing away from the foot skeleton.

Optionally, the force equalizing plate is integrally formed, the force equalizing plate includes a fore sole portion corresponding to a front end of the foot skeleton, a rear sole portion corresponding to a rear end of the foot skeleton, and a foot arch connected between the fore sole portion and the rear sole portion, the fore sole portion, the rear sole portion and the foot arch are integrally formed, and the fore sole portion and the rear sole portion are both provided with the flexible pad; or the force equalizing plate includes a fore sole portion and a rear sole portion separated from each other, the fore sole portion corresponds to a front end of the foot skeleton and is connected to the foot skeleton, the rear sole portion corresponds to a rear end of the foot skeleton and is connected to the foot skeleton, and the fore sole portion and the rear sole portion are both provided with the flexible pad.

In a third aspect, embodiments of the present invention provide a robotic leg, the robotic leg includes a leg structure and the foot structure as described in the second aspect, and the foot structure is connected to the leg structure.

In a fourth aspect, embodiments of the present invention provide a legged robot, and the legged robot includes at least one robotic leg as described in the third aspect.

Technical solutions provided by the embodiments of the present invention may include the following beneficial effects.

In the foot skeleton of the present invention, the force detection unit is arranged at a position of the at least one deformation arm, so that the force path of the foot sole is changed, and the external force applied to the foot sole may be transmitted to each force detection unit, so as to enhance the sensing accuracy of the force detection unit, thus simplifying the structural difficulty, improving the integration level and the space utilization rate of the foot structure, and reducing the volume and weight of the structure.

It should be understood that the above general description and the following detailed description are illustrative and explanatory only, and do not limit the present invention.

### BRIEF DESCRIPTION OF THE DRAWINGS

The drawings are incorporated into the specification and form a part of the specification, illustrate embodiments consistent with the present invention, and are used in conjunction with the specification to explain the principles of the present invention.
Fig. 1 is a perspective view of a foot structure according to an illustrative embodiment of the present invention.
Fig. 2 is a front view of the foot structure Fig. 1.
Fig. 3 is a top view of the foot structure Fig. 1.
Fig. 4 is a side view of the foot structure Fig. 1.
Fig. 5 is a perspective view of a foot skeleton according to an illustrative embodiment of the present invention.
Fig. 6 is a front view of the foot skeleton Fig. 5.
Fig. 7 is a top view of the foot skeleton Fig. 5.
Fig. 8 is a side view of the foot skeleton Fig. 5.
Fig. 9 is a perspective view of a foot structure according to another illustrative embodiment of the present invention.
Fig. 10 is an exploded view of the foot structure Fig. 9.
Fig. 11 is a perspective view of a foot structure according to another illustrative embodiment of the present invention.
Fig. 12 is an exploded view of the foot structure Fig. 11.
Fig. 13 is a front view of the foot structure Fig. 11.
Fig. 14 is an enlarged view of portion A in Fig. 13.
Fig. 15 is a perspective view of a foot structure according to another illustrative embodiment of the present invention.
Fig. 16 is an exploded view of the foot structure Fig. 15.
Fig. 17 is a front view of the foot structure Fig. 15.
Fig. 18 is an enlarged view of portion B in Fig. 17.
Fig. 19 is a perspective view of a foot structure according to another illustrative embodiment of the present invention.

### DETAILED DESCRIPTION

In order to better understand the technical solution of the present invention, a foot skeleton, a foot structure, a robotic leg, and a legged robot of the present invention are described in detail below with reference to the drawings. The features in the following embodiments and implementations may be combined with each other without conflict.

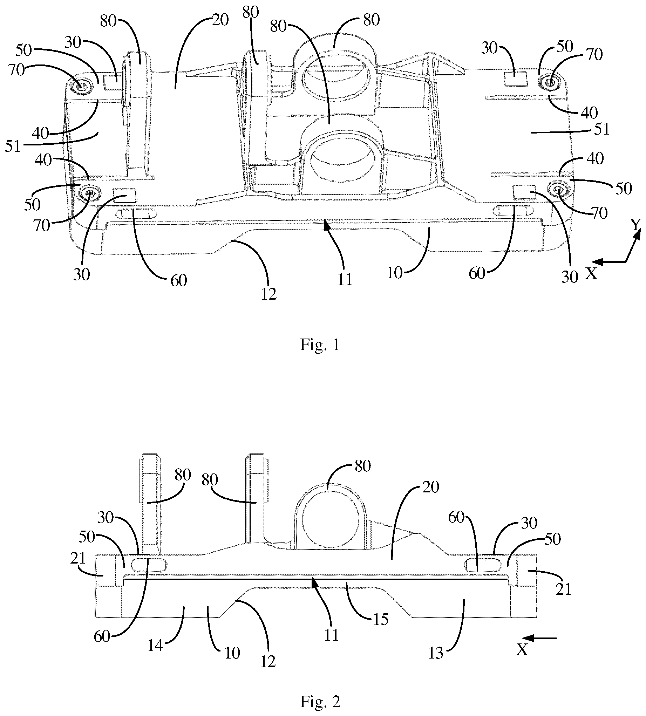

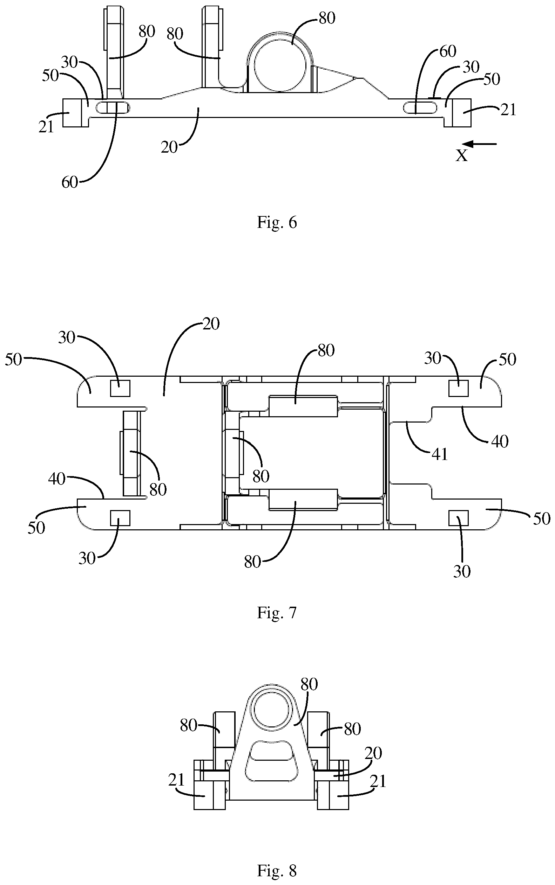

Referring to Figs. 1 to 8, an embodiment of the present invention present provides a foot structure, and the foot structure may be applicable to a bipedal robot or other types of legged robots for detecting a force condition of a foot sole of a robot. The foot structure may include: a force equalizing plate 10, a foot skeleton 20 and a plurality of force detection units 30. The foot skeleton 20 is connected to the force equalizing plate 10. The foot skeleton 20 is provided with at least one deformation arm 50, 52, the at least one deformation arm 50, 52 is arranged along at least one of a length direction X and a width direction Y of the foot skeleton 20, and a side portion of the at least deformation arm 50, 52 is provided with a deformation hole 60. The at least one deformation arm 50, 52 is provided with at least one force detection unit 30. In some embodiments, the number of the force detection units 30 may correspond to the number of the deformation arms 50, 52 and the force detection units 30 may be arranged in a one-to-one correspondence to the deformation arms 50, 52. It may be understood that each deformation arm 50 or 52 may be provided with one or more force detection units 30, or no force detection unit. Further, the number of the force detection units 30 arranged to each deformation arm 50 or 52 which is provided with the force detection unit 30 may be the same or different, which is not limited in the present invention.

In some embodiments, in an example shown in Figs. 1 and 2, a left end portion of the foot skeleton 20 along the length direction X may refer to a rear end, a right end portion of the foot skeleton 20 along the length direction X may refer to a front end, and each of the front end and the rear end is provided with at least one deformation arm 50, 52 and at least one force detection unit 30, so as to ensure that the force condition of the front and rear ends of the entire foot structure may be detected, thus improving the accuracy of detection.

It may be understood that the deformation hole 60 may determine the strain capacity of the deformation arm 50 or 52, and the larger the deformation hole 60 is, the greater a strain degree (i.e., a degree of deformation) of the deformation arm 50 or 52 is, when subjected to the same force. An external force applied to the foot sole may be transmitted to a position of each force detection unit 30 through the force equalizing plate 10, thereby causing the deformation of the deformation arm 50 or 52. The force detection unit 30 and the deformation arm 50 or 52 are tightly attached together to synchronously generate deformation, so that the strain degree of the deformation arm 50 or 52 may be reflected and obtained through detecting a strain degree of the force detection unit 30, so as to obtain the force condition of the entire foot structure. In some embodiments, the force detection unit 30 may include a stress patch or other forms of force sensors, the stress patch may be understood as a small-sized flexible circuit board (FPC), in which copper wires crisscrossed are provided, and the stress patch and the deformation arm 50 or 52 are tightly attached to each other. When the foot structure is subjected to an external force, the deformation arm 50 or 52 and the stress patch are able to generate deformation synchronously, and the copper wires of the stress patch are stretched and extruded by an action of the external force, resulting in changes of a voltage and an electrical resistance, so that by detecting the change condition of the voltage value, which may be understood as by obtaining the force condition at the deformation arm through detection and calculation based on the calibration fusion algorithm, the deformation degree of the deformation arm may be reflected and obtained, so as to obtain the force condition of the foot sole of the entire foot structure.

It may be known from the above technical solution that, in the foot structure of the present invention, the force detection unit 30 is arranged at the position of the at least one deformation arm 50 or 52, so that the force path of the foot sole is changed, and thus the external force applied to the foot sole may be transmitted to the position of each force detection unit 30 through the force equalizing plate 10. The strain degree at the deformation arm 50 or 52 is detected by the force detection unit 30, and then the force at the deformation arm is obtained by the calibration fusion algorithm, so as to obtain the force condition of the foot sole to enhance the sensing accuracy of the force detection unit 30, thus simplifying the structural difficulty, improving the integration level and the space utilization rate of the foot structure, and reducing the volume and weight of the structure.

In some embodiments, the foot skeleton 20 is provided with a plurality of grooves 40, 42, the grooves are arranged along at least one of the length direction X and the width direction Y of the foot skeleton 20, the foot skeleton 20 is separated by the grooves to form a plurality of deformation arms 50, 52. In some embodiments, each of two ends of the foot skeleton 20 along the length direction X is provided with at least one groove, the groove extends inwardly from an end edge of the foot skeleton 20 along the length direction X. Each of the two ends of the foot skeleton 20 along the length direction X is separated by the groove to form at least one deformation arm, and the side portion of the deformation arm is provided with at least one deformation hole 60.

In some embodiments, each of the two ends of the foot skeleton 20 along the length direction X is provided with a first groove 40, each of the two ends of the foot skeleton 20 along the length direction X is separated by the first groove 40 to form a plurality of first deformation arms 50. In other examples, each of two ends of the foot skeleton 20 along the width direction Y is provided with a second groove 42, and each of the two ends of the foot skeleton 20 along the width direction Y is separated by the second groove 42 to form a plurality of second deformation arms 52.

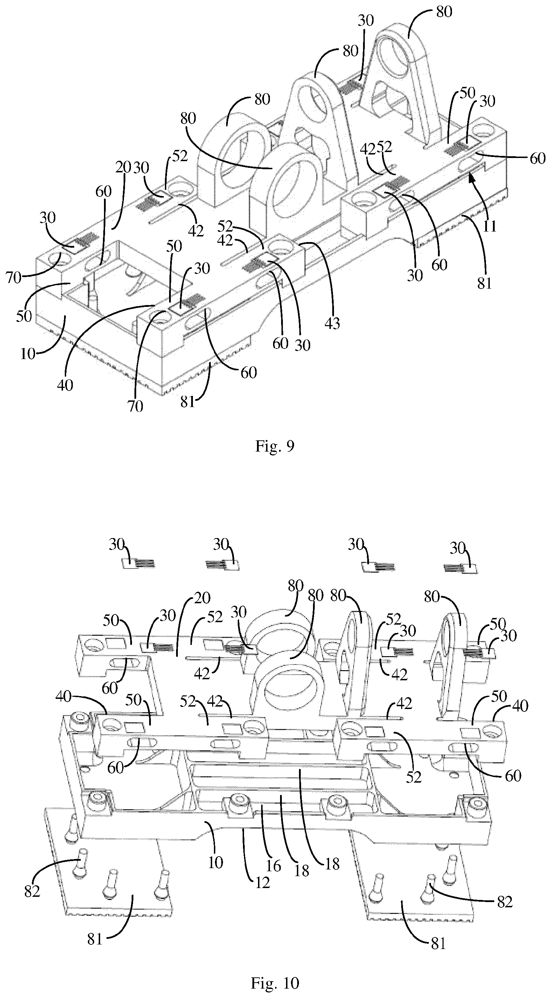

For example, referring to the examples shown in Figs. 1 to 8, each of the two ends of the foot skeleton 20 along the length direction X is provided with the first groove 40. In the example shown in Figs. 9 and 10, each of the two ends of the foot skeleton 20 along the length direction X is provided with the first groove 40, and each of the two ends of the foot skeleton 20 along the width direction Y is also provided with the second groove 42.

In some embodiments, a forming manner of the first groove 40 may include the following two.

(1) As shown in Fig. 1, the first groove 40 is in a shape of a slit, each of the two ends of the foot skeleton 20 along the length direction X is provided two first grooves 40, and the two first grooves 40 located at the same side of the foot skeleton 20 are spaced apart along the width direction Y of the foot skeleton 20. Each of the two ends of the foot skeleton 20 along the length direction X is separated by the two first grooves 40 located at the same side to form two first deformation arms 50 and a main body portion 51 located between the two first deformation arms 50. That is, the foot skeleton 20 is provided with four first deformation arms 50.

It may be understood that the first deformation arms 50 are configured to generate deformation under the influence of the external force, the force condition of the entire foot structure may be reflected and obtained through the synchronized deformations of the force detection unit 30 and the first deformation arm 50, and the main body portion 51 does not generate deformation under the influence of the external force, so that the overall structural strength of the foot skeleton 20 and the foot structure can be improved. In some embodiments, four first deformation arms 50 and four force detection units 30 are arranged at four corners of the foot skeleton 20, respectively. As such, the force condition of the foot sole of the entire foot structure is expressed by a resultant force measured by the four force detection units 30 located at the four corners, thus improving the accuracy of the detection. It is to be noted that the number of the first grooves 40 and the number of the first deformation arms 50 may be set according to the actual situation, which is not limited in the present invention.

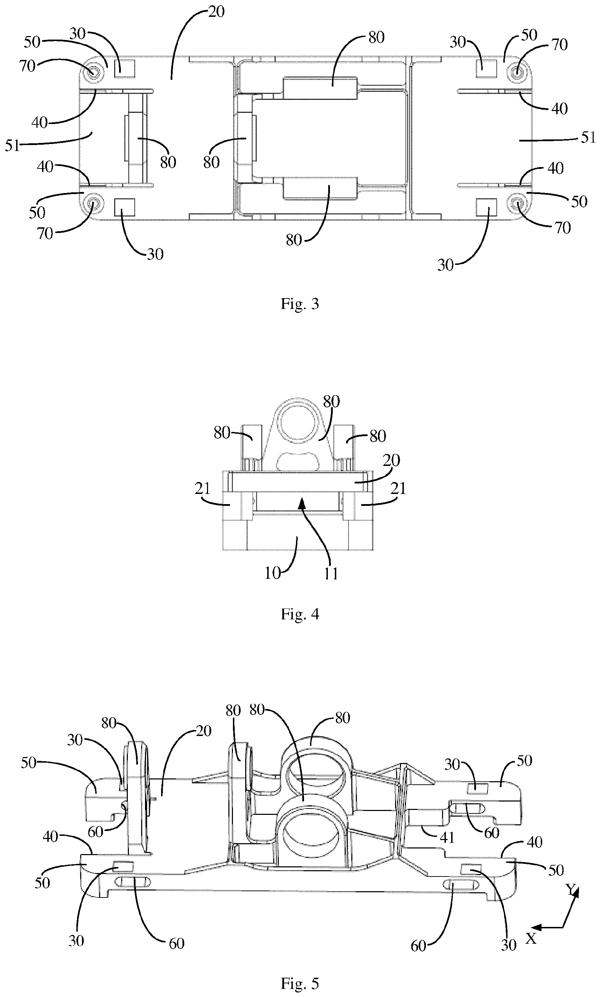

(2) As shown in Fig. 5, the first groove 40 has a rectangular shape, each of the two ends the foot skeleton 20 along the length direction X is provided with one first groove 40, and each of the two ends of the foot skeleton 20 is separated by the first groove 40 to form two first deformation arms 50. That is, the foot skeleton 20 is provided with four first deformation arms 50.

It may be understood that the first deformation arms 50 are configured to generate deformation under the influence of the external force, and the force condition of the entire foot structure may be reflected and obtained through the synchronized deformations of the force detection unit 30 and the first deformation arm 50, and the overall structural strength of the foot skeleton 20 and the foot structure can be improved. In some embodiments, four first deformation arms 50 and four force detection units 30 are arranged at four corners of the foot skeleton 20, respectively. As such, the force condition of the foot sole of the entire foot structure is expressed by a resultant force measured by the four force detection units 30 located at the four corners, thus improving the accuracy of the detection. It is to be noted that the number of the first grooves 40 and the number of the first deformation arms 50 may be set according to the actual situation, which is not limited in the present invention. It is to be understood, compared to the example shown in Figs. 1 to 4, the example shown in Figs. 5 to 8 is equivalent to digging out the main body portion 51 in the example shown in Figs. 1 to 4, thus achieving a weight reduction effect.

Further, the foot skeleton 20 is further provided with a weight reduction groove 41 at a position adjacent to the first groove 40, and the weight reduction groove 41 has a rectangular shape and in communication with the first groove 40. Along the width direction Y of the foot skeleton 20, a length of the weight reduction groove 41 is less than a length of the first groove 40. A weight reduction effect may be further achieved. In some embodiments, the weight reduction groove 41 and the first groove 40 are combined to form a rectangular groove, and a circular arc transition structure is formed at a connection position of the weight reduction groove 41 and the first groove 40, which further facilitates processing and molding.

In some embodiments, two first grooves 40 located at the same side of the foot skeleton 20 along the length direction X are symmetrically arranged with respect to a center line along the length direction X of the foot skeleton 20. Two first grooves 40 located at the same side of the foot skeleton 20 along the width direction Y are symmetrically with respect to a center line arranged along the width direction Y of the foot skeleton 20.

Accordingly, two first deformation arms 50 located at the same side of the foot skeleton 20 along the length direction X are symmetrically arranged along with respect to the center line the length direction X of the foot skeleton 20. Two first deformation arms 50 located at the same side of the foot skeleton 20 along the width direction Y are symmetrically arranged with respect to the center line along the width direction Y of the foot skeleton 20.

As such, the first deformation arms 50 are uniformly distributed and formed at the four corners of the foot skeleton 20 and the force detection units 30 are uniformly arranged at the four corners of the foot skeleton 20, the external force applied to the foot sole may be uniformly transmitted to each force detection unit 30 by the force equalizing plate 10, and the force condition of the foot sole of the entire foot structure may be expressed by the resultant force measured by the four force detection units 30 located at the four corners. That is, the strain degree at the first deformation arm 50 may be detected by the force detection unit 30, and then the force at the deformation arm may be obtained by the calibration fusion algorithm, so as to obtain the force condition of the foot sole, thus improving the sensing accuracy of the force detection unit 30. Therefore, the force condition of the foot sole of the entire foot structure may be more accurately detected, and the accuracy of the detection is improved.

Referring to Figs. 9 and 10, in this embodiment, a forming manner of the first groove 40 may be the same as the forming manner of the embodiment of Figs. 1 to 4 described above, and also may be the same as the forming manner of the embodiment of Figs. 5-8 described above. A forming manner of the second groove 42 may be as follows: each of the two ends of the foot skeleton 20 along the width direction Y is provided with a notch portion 43 and two second grooves 42 in communication with the notch portion 43, and the two second grooves 42 located at the same side of the foot skeleton 20 are spaced apart along the length direction X of the foot skeleton 20. Each of the two ends of the foot skeleton 20 along the width direction Y is separated by the two second grooves 42 located at the same side to form two second deformation arms 52. That is, the foot skeleton 20 is provided with four first deformation arms 50 and four second deformation arms 52, for a total of eight deformation arms.

In some embodiments, an axial direction of the deformation hole 60 extends along a width direction of the deformation arm, i.e. extends along the width direction Y of the foot skeleton 20. In the examples shown in Figs. 1 to 10, a section of the deformation hole 60 in a direction perpendicular to the axial direction of the deformation hole 60 is in a shape of a ring. In some embodiments, the deformation hole 60 is in a shape of a runway circle, which facilitates processing and molding, and provides a better deformation space and a better deformation capacity for the deformation arm 50 or 52 to improve the detection effect. It should be noted that the shape of the deformation hole 60 may also be configured as other shapes according to actual needs, which is not limited in the present invention.

It may be understood that the length of the first groove 40 along the length direction of the foot skeleton 20 determines a length of the first deformation arm 50 along the length direction of the foot skeleton 20. The longer the length of the first groove 40 along the length direction of the foot skeleton 20 is, the greater the deformation capacity of the first deformation arm 50 is. The shorter the length of the first groove 40 along the length direction of the foot skeleton 20 is, the smaller the deformation capacity of the first deformation arm 50 is. A length of the first groove 40 along the width direction of the foot skeleton 20 determines a length of the first deformation arm 50 along the width direction of the foot skeleton 20. The longer the length of the first groove 40 along the width direction of the foot skeleton 20 is, the smaller the deformation capacity of the first deformation arm 50 is. The shorter the length of the first groove 40 along the width direction of the foot skeleton 20 is, the larger the deformation capacity of the first deformation arm 50 is. Similarly, the second groove 42 and the second deformation arm 52 also have the same corresponding relationship.

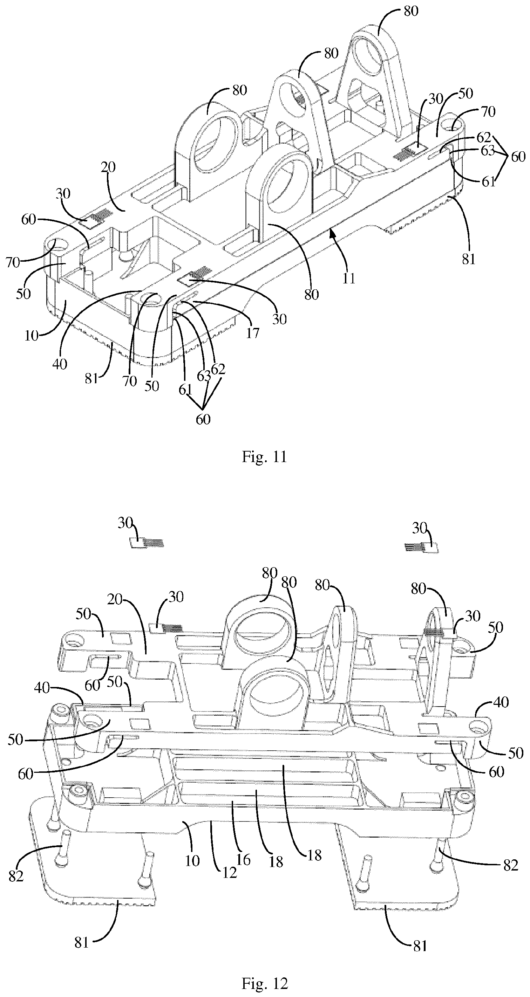

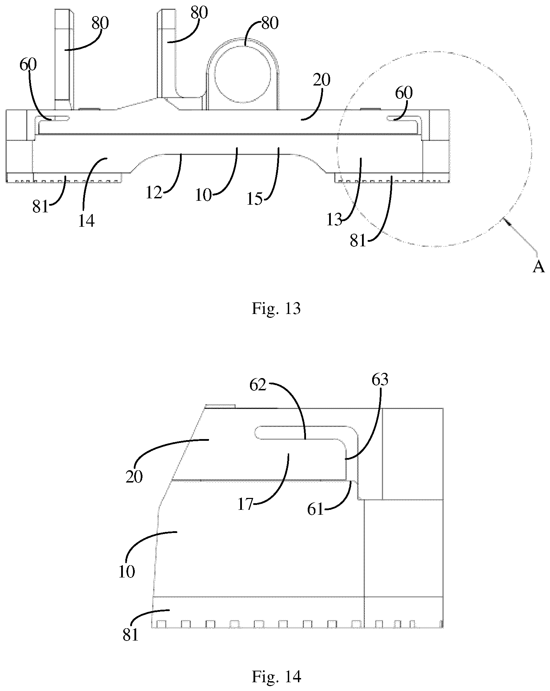

As shown in Figs. 11 to 14, the deformation hole 60 includes an opening end 61, the opening end 61 runs through a bottom surface of the deformation arm (i.e. the foot skeleton 20). In some embodiments, the deformation hole 60 includes a first hole portion 62 and a second hole portion 63 in communication with the first hole portion 62, and the second hole portion 63 includes the opening end 61. An axial direction of the first hole portion 62 and an axial direction of the second hole portion 63 both extend along the width direction of the deformation arm (i.e., the width direction Y of the foot skeleton 20). The first hole portion 62 and the second hole portion 63 are connected by a bent portion. The second hole portion 63 runs through the bottom surface of the deformation arm (i.e. the foot skeleton 20) from the opening end 61. In some embodiments, a bent angle between the first hole portion 62 and the second hole portion 63 may be between 75 degrees and 105 degrees.

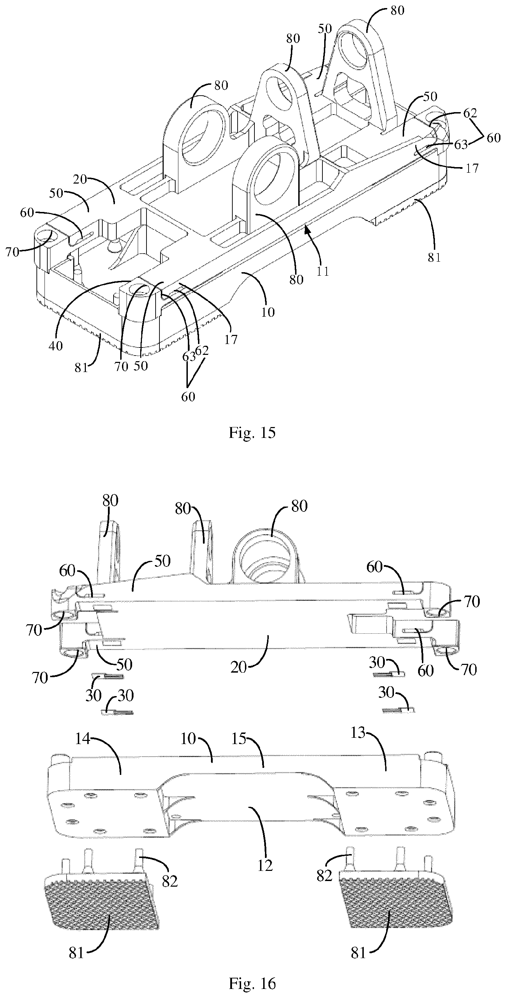

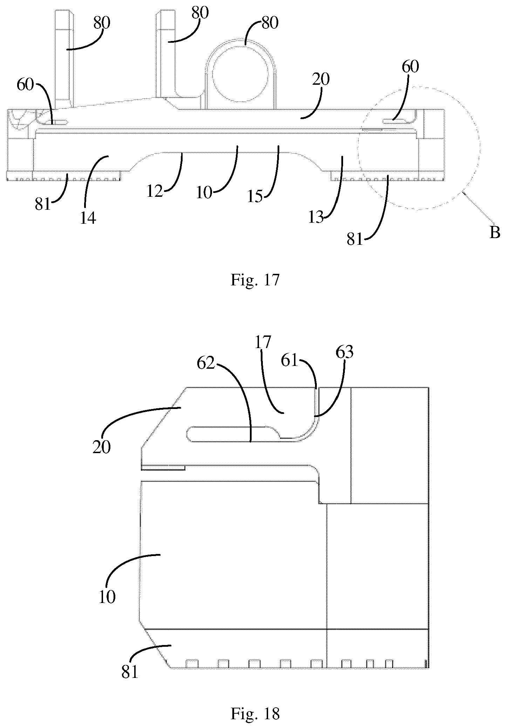

As shown in Figs. 15 to 18, the deformation hole 60 includes an opening end 61, the opening end 61 runs through a top surface of the deformation arm (i.e. the foot skeleton 20). In some embodiments, the deformation hole 60 includes a first hole portion 62 and a second hole portion 63 in communication with the first hole portion 62, and the second hole portion 63 includes the opening end 61. An axial direction of the first aperture portion 62 and an axial direction of the second aperture portion 63 both extend along the width direction of the deformation arm (i.e., the width direction Y of the foot skeleton 20), and the first hole portion 62 and the second hole portion 63 are connected by a bent portion. The second hole portion 63 runs through the top surface of the deformation arm (i.e. the foot skeleton 20) from the opening end 61.

In some embodiments, a diameter of the first hole portion 62 is the same as a diameter of the second hole portion 63, as in the example shown in Fig. 11. In the case of process condition allowance, two side portions of either end of the foot skeleton 20 along the length direction X may be simultaneously provided with holes by a diamond wire, so that the first deformation arms 50 at the same side of the foot skeleton 20 are provided with the deformation hole 60, respectively.

In some embodiments, the diameter of the first hole portion 62 is larger than the diameter of the second hole portion 63, as in the example shown in Fig. 15. In the case of process condition allowance, two side portions of either end of the foot skeleton 20 along the length direction X may be simultaneously provided holes by a diamond wire, and when encountering a corresponding portion of the foot skeleton 20 corresponding to a mounting portion 80 for connecting to a leg structure, the diamond wire needs to avoid the mounting portion 80. That is, the diamond wire may form the second hole portion 63 first until being adjacent to the corresponding portion under the mounting portion 80, and then a milling process manner may replace the diamond wire and be used for forming the first hole portion 62 with a relatively larger diameter.

In some embodiments, the first hole portion 62 and the second hole portion 63 are perpendicular to each other, i.e., the deformation hole 60 is L-shaped. Further, the bent portion between the first hole portion 62 and the second hole portion 63 is a circular arc transition structure. As such, when the foot skeleton 20 is subjected to an external force, inner walls at the deformation arms may be in contact with each other in a larger area, and the larger the contact area is, the more accurate the detected force condition is, thus improving the accuracy of the force detection.

It may be understood that when the second hole portion 63 runs through the bottom surface of the deformation arm, a protective structure 17 may be formed at a lower portion of each deformation arm, and the protective structure 17 is configured to bear a great pressure. When the second hole portion 63 runs through the top surface of the deformation arm, a protective structure 17 may be formed at an upper portion of each deformation arm, and the protective structure is configured to subject a great pressure. When the deformation arm is subjected to a pressure to generate a deformation and the deformation exceeds a certain threshold value, the protective structure will prevent the deformation arm from continuing to be deformed, thus realizing an overload protection function.

It may be understood, as in the example shown in Figs. 11 to 14, the opening end 61 of the deformation hole 60 downwardly runs through the deformation arm. When the deformation arm is subjected to a pressure from above, the deformation arm is downwardly deformed. When the deformation exceeds a certain threshold value, the protection structure 17 will prevent the deformation arm from continuing to be deformed, thus realizing the overload protection function. When the deformation arm is subjected to a pressure from below, the deformation arm is downwardly deformed due to a gap 11 defined between the force equalizing plate 10 and the foot skeleton 20, and when the deformation exceeds a certain threshold value, the protective structure 17 will contact the force equalizing plate 10 until upside and downside inner walls of the first hole portion 62 contact with each other, so that the force equalizing plate is prevented from continuing to exert an upward pressure to the deformation arm, and the deformation arm is prevented from continuing to be deformed, thus realizing the overload protection function.

It may be understood, as in the example shown in Figs. 15 to 18, the opening end 61 of the deformation hole 60 upwardly runs through the deformation arm. When the deformation arm is subjected to a pressure from above, the deformation arm is downwardly deformed. When the deformation exceeds a certain threshold value, the protective structure 17 is downwardly deformed until upside and downside inner walls of the first hole portion 62 contact with each other, so that the deformation arm is prevented from continuing to be deformed, thus realizing the overload protection function.

In some embodiments, each of two ends of the deformation hole 60 along the axial direction runs through sidewalls at two sides of the deformation arm 50 or 52 along the width direction Y, i.e., the deformation hole 60 is an open hole with two ends both running through the deformation arm 50 or 52. In some embodiments, at least one end of the two ends of the deformation hole 60 along the axial direction runs through a sidewall of the deformation arm 50 or 52 along the width direction Y, i.e., the deformation hole 60 is a semi-closed hole with one end running through the deformation arm 50 or 52. In some embodiments, neither end of the deformation hole 60 along the axial direction runs through sidewalls at two sides of the deformation arm 50 or 52 along the width direction Y, i.e., the deformation hole 60 is a closed hole with two ends not running through the deformation arm 50 or 52. It should be noted that the type of the deformation hole 60 may be set according to actual needs, which is not limited in the present invention.

In some embodiments, the force detection unit 30 is arranged at a position of a maximum strain of the deformation arm 50 or 52. As such, the deformation arm 50 or 52 may achieve deformation with a great degree, so that the force condition in a great range of the foot sole can be detected, thus improving the detection performance. It is to be noted that the position of the deformation hole 60 determines the position of the maximum strain of the deformation arm 50 or 52, and it is necessary that the force detection unit 30 is arranged at the position of the maximum strain. The position of the deformation holes 60 may be set according to actual needs, which is not limited in the present invention.

It is to be noted that the first groove 40 extends along the length direction X of the foot skeleton 20, the axial direction of the deformation hole 60 extends along the width direction of the deformation arm 50 or 52, and such an arrangement manner of the first groove 40 and the deformation hole 60 may provide a better deformation space and a better deformation capacity for the deformation arm 50 or 52, thus improving the detection effect.

In addition to the embodiments described above, in other examples, the first groove 40 may also extend along the width direction Y of the foot skeleton 20, and the deformation hole 60 may extend along a length direction of the first deformation arm 50 (i.e. the length direction X of the foot skeleton 20). In some embodiments, the first groove 40 may also extend along the width direction Y of the foot skeleton 20, and the deformation hole 60 may extend along a width direction of the first deformation arm 50 (i.e. the width direction Y of the foot skeleton 20). In some embodiments, the first groove 40 may also extend along the length direction X of the foot skeleton 20, and the deformation hole 60 may extend along a length direction of the first deformation arm 50 (i.e. the length direction X of the foot skeleton 20). The arrangement manner of the first groove 40 and the deformation hole 60 may be set according to actual needs, which is not limited in the present invention.

In some embodiments, the foot structure may further include a plurality of fasteners 70, the fastener 70 passes through the deformation arm 50 or 52 and is detachably connected to the force equalizing plate 10, so that the mutual fixation of the foot skeleton 20 and the force equalizing plate 10 is realized, and also the detachable connection of the foot skeleton 20 and the force equalizing plate 10 is realized. Portions of the foot skeleton 20 and the force equalizing plate 10 corresponding to the fastener 70 abut against each other and the gap 11 is formed between the remaining portions of the foot skeleton 20 and the force equalizing plate 10. In some embodiments, the fastener 70 may be a screw or another connecting member.

In some embodiments, the force equalizing plate 10 is an integrally formed structure, the force equalizing plate 10 includes a fore sole portion 13 corresponding to the front end of the foot skeleton 20 (i.e., a right end region of the foot skeleton), a rear sole portion 14 corresponding to the rear end of the foot skeleton 20 (i.e., a left end region of the foot skeleton), and a foot arch 15 connected between the fore sole portion 13 and the rear sole portion 14. The foot arch 15 corresponds to a middle region of the foot skeleton 20, and the foot arch 15 may increase the adaptability of the foot sole to different surfaces. The fore sole portion 13, the rear sole portion 14, and the foot arch 15 are integrally formed.

In some embodiments, the force equalizing plate 10 and the foot skeleton 20 are integrally formed or connected to each other by the fasteners 70 such as screws. Portions of the foot skeleton 20 and the force equalizing plate 10 corresponding to the force detection unit 30 abut against each other, and the gap 11 is formed between the remaining portions of the foot skeleton 20 and the force equalizing plate 10.

As such, it may be ensured that the external force applied to the foot sole may be transmitted by the force equalizing plate 10 to each force detection unit 30, and the force applied to the foot may be completely transmitted to the four deformation arms 50, 52. That is, the force equalizing plate 10 is connected to the foot skeleton 20 by the fasteners 70 at the deformation arms 50, 52, and does not contact with the remaining portions of the foot skeleton 20, so as to ensure that the total force applied to the foot is transmitted to the deformation arms 50, 52 by the force equalizing plate 10. Then, the strain degree at the deformation arm 50 or 52 is detected by the force detection unit 30, and then the force at the deformation arm is obtained by the calibration fusion algorithm so as to obtain the force condition of the foot sole, thus enhancing the sensing accuracy of the force detection unit 30 and ensuring the accuracy of the measurement.

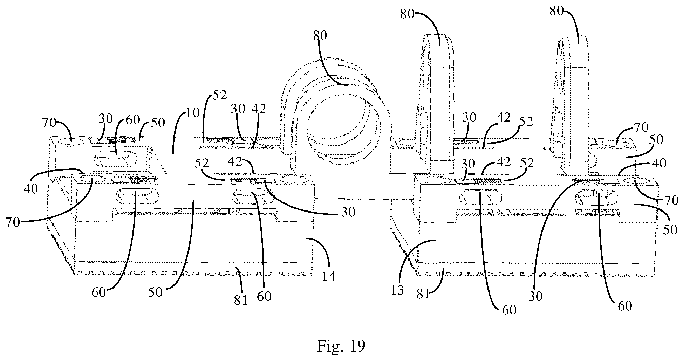

Referring to Fig. 19, in some other embodiments, the force equalizing plate 10 includes a fore sole portion 13 and a rear sole portion 14 which are separated, the fore sole portion 13 corresponds to the front end of the foot skeleton 20 and is connected to the foot skeleton 20, and the rear sole portion 14 corresponds to the rear end of the foot skeleton 20 and is connected to the foot skeleton 20. That is, the force equalizing plate adopts a split structural type.

In some embodiments, a region of the foot skeleton 20 corresponding to the fore sole portion 13 is provided with a plurality of deformation arms, which are spaced apart from each other, along a circumferential direction of the fore sole portion 13. A region of the foot skeleton 20 corresponding to the rear sole portion 14 is provided with a plurality of the deformation arms, which are spaced apart from each other, along the circumferential direction of the rear sole portion 14. As such, the deformation arms are distributed in the circumferential directions of both the fore sole portion and the rear sole portion, so that the respective force conditions of both the fore sole portion and the rear sole portion can be detected more accurately. In some embodiments, each of four corners of each of the fore sole portion and the rear sole portion is provided one deformation arm, i.e. each of the fore sole portion and the rear sole portion is provided with four deformation arms.

It may be understood that the force equalizing plate adopts the split structural type, i.e., the fore sole portion and the rear sole portion are separated, so that it is possible for fore sole portion and the rear sole portion to independently sense the position of contacting the ground. Thus, compared to the above force equalizing plate of the integrally formed foot sole structural type, it is possible to more accurately judge the situation of both the fore sole portion and the rear sole portion contacting the ground. For example, it is possible to more accurately distinguish a situation in which the whole foot falls to the ground and a situation in which the center of the foot sole steps on the gravel. It may be satisfied that the force equalizing plate may transmit the force upwardly through each point where each deformation arm is arranged, when the foot sole falls to the ground in different postures, or when the foot sole steps on a stone, a step and other objects in different positions. In addition, the force equalizing plate adopts the split structural type, and the fore sole portion and the rear sole portion are separated, so that when the foot sole contacts the ground in different positions, different force values are transmitted at the 4 points, and the contacting position of the foot sole may be analyzed by calculation, so as to provide a reference for the action posture correction of the robot. When the robot adopting this foot structure walks on uneven road surface, the position of the foot sole contacting the ground can be better analyzed.

Further, the middle of the foot arch 15 is provided a hollow-out portion 16, and a reinforcing rib 18 is arranged in the hollow-out portion 16 for increasing the rigidity of the force equalizing plate. The force equalizing plate is connected to the foot skeleton by a fastener such as a screw etc. When the force equalizing plate contacts the ground, the acting force is transmitted to each deformation arm of the foot skeleton by the four corners of each of the fore sole portion and rear sole portion. In a case that the rigidity of the force equalizing plate is not enough, the data of a strain gauge is different when the foot sole steps on the ground (carpet, marble ground) with different hardness (soft or hard), thus resulting in calculating a different force value. Therefore, the reinforcing rib can enhance the rigidity of the force equalizing plate, and can improve the adaptability of the foot sole to different grounds.

In some embodiments, the foot structure may also include a flexible pad 81, the flexible pad 81 is arranged at a side of the force equalizing plate 10 facing away from the foot skeleton 20, the flexible pad 81 may be used to cushion the foot sole falling on the ground to reduce the transient impact, and may also enhance the contacting friction between the foot structure and the ground, thus increasing the stability. In some embodiments, the flexible pad 81 may be a rubber pad. The rubber pad is affixed under the force equalizing plate 10, and the rubber pad is connected to a metal surface of the force equalizing plate using a connecting member 82 such as a mushroom nail and glue. The force equalizing plate is connected to the foot skeleton by a fastener such as a screw etc. When the force equalizing plate contacts the ground, the acting force is transmitted to each deformation arm of the foot skeleton by the four corners of each of the fore sole portion and rear sole portion. In some embodiments, when the force equalizing plate adopts the split structural type, both the fore sole portion and the rear sole portion are provided with the flexible pad 81.

It is to be noted that, in order to define the gap 11 described above, the portion of the foot skeleton 20 corresponding to the deformation arm 50 or 52 may project towards the equalizing plate 10 to form a projection 21, so that the bottom of the foot skeleton 20 (i.e., a side of the foot skeleton 20 adjacent to the force equalizing plate 10) is provided with a groove, so that when the foot skeleton 20 and the force equalizing plate 10 are assembled to each other, the gap 11 may be formed at this groove between the foot skeleton 20 and the force equalizing plate 10.

In some embodiments, a side of the force equalizing plate 10 facing away from the foot skeleton 20 is provided with a recessed portion 12. When the foot structure walks on an uneven road, the recessed portion 12 may be used to accommodate obstacles, so as to provide an obstacle avoidance function, and ensure the balance of the foot structure, thus expanding the applicability of the legged robot. In some embodiments, the recessed portion 12 is located at the middle of the force equalizing plate 10, i.e., corresponding to the position of the foot arch, so that when the legged robot steps on an obstacle, the forces applied to the two ends of the foot sole are more equalized, thus ensuring the balance of the foot structure.

In some embodiments, the foot skeleton 20 may also be provided with a plurality of mounting portions 80 spaced part from each other, and the foot structure may be assembled and connected to other structural parts of the robot, such as a leg structure, by the mounting portions 80. In this embodiment, each of the two ends of the foot skeleton 20 along the length direction X is provided with one mounting portion 80, and each of the two ends of the foot skeleton 20 along the width direction Y is also provided with one mounting portion 80. It is to be noted that the positions and the number of the mounting portions 80 may be set according to the actual situation, which is not limited in the present invention.

Embodiments of the present invention also provide a robotic leg applicable to a legged robot. The robotic leg may include a leg structure and at least one foot structure, and the foot structure is connected to the leg structure. It is to be noted that the descriptions of the foot structure in the above embodiments and implementations are also applicable to the robotic leg of this embodiment. The foot structure may be movably connected to the leg structure.

The robotic leg adopts the foot structure of the above embodiments of the present invention, the foot skeleton 20 is separated by the grooves to form the deformation arms, and the force detection unit 30 is arranged at the position of at least one deformation arm, so that the force path of the foot sole is changed, the external force applied to the foot sole may be transmitted by the force equalizing plate 10 to each force detection unit 30, the strain degree at the deformation arm is detected by the force detection unit 30, and then the force at the deformation arm is obtained by the calibration fusion algorithm, so as to obtain the force condition of the foot sole, thus improving the sensing accuracy of the force detection unit 30, simplifying the structural difficulty, improving the integration level and the space utilization rate of the foot structure, and reducing the volume and weight of the overall structure of the legged robot.

Embodiments of the present invention also provide a legged robot, the legged robot includes at least one robotic leg. It is to be noted that the descriptions of the foot structure and the robotic leg in the above embodiments and implementations are also applicable to the legged robot of this embodiment. In some embodiments, the legged robot may be a bipedal robot or another legged robot with another number of legs.

The legged robot adopts the foot structure of the above embodiments of the present invention, the foot skeleton 20 is separated by the grooves to form the deformation arms, and the force detection unit 30 is arranged at the position of at least one deformation arm, so that the force path of the foot sole is changed, the external force applied to the foot sole may be transmitted by the force equalizing plate 10 to each force detection unit 30, the strain degree at the deformation arm is detected by the force detection unit 30, and then the force at the deformation arm is obtained by the calibration fusion algorithm, so as to obtain the force condition of the foot sole, thus improving the sensing accuracy of the force detection unit 30, simplifying the structural difficulty, improving the integration level and the space utilization rate of the foot structure, and reducing the volume and weight of the overall structure of the legged robot.

It should be understood that the present invention is not limited to the precise structure that has been described above and illustrated in the drawings, and that various modifications and changes may be made without departing from the scope of the present invention. The scope of the present invention is limited only by the appended claims.

## Claims

1. A foot skeleton (20), comprising at least one deformation arm (50, 52), wherein the at least one deformation arm (50, 52) is arranged along at least one of a length direction and a width direction of the foot skeleton (20), a side portion of the at least one deformation arm (50, 52) comprises a deformation hole (60), and the at least one deformation arm (50, 52) is provided with at least one force detection unit (30).

2. The foot skeleton (20) according to claim 1, wherein
the foot skeleton (20) comprises a plurality of grooves (40, 42);
the plurality of grooves (40, 42) are arranged along at least one of the length direction and the width direction of the foot skeleton (20); and
the foot skeleton (20) is separated by the plurality of grooves (40, 42) to form a plurality of deformation arms (50, 52).

3. The foot skeleton (20) according to claim 2, wherein each of two ends of the foot skeleton (20) along the length direction comprises a first groove (40), and each of the two ends of the foot skeleton (20) along the length direction is separated by the first groove (40) to form a plurality of first deformation arms (50); and/or
each of two ends of the foot skeleton (20) along the width direction comprises a second groove (42), and each of the two ends of the foot skeleton (20) along the width direction is separated by the second groove (42) to form a plurality of second deformation arms (52).

4. The foot skeleton (20) according to claim 3, wherein
the first groove (40) is in a shape of a slit;
each of the two ends of the foot skeleton (20) along the length direction comprises two first grooves (40), the two first grooves (40) located at the same side of the foot skeleton (20) are spaced apart along the width direction of the foot skeleton (20); and
each of the two ends of the foot skeleton (20) along the length direction is separated by the two first grooves (40) located at the same side to form two first deformation arms (50) and a main body portion (51) located between the two first deformation arms (50),
preferably, the two first grooves (40) located at the same side along the length direction of the foot skeleton (20) are symmetrically arranged with respect to a center line along the length direction of the foot skeleton (20); the two first grooves (40) located at the same side along the width direction of the foot skeleton (20) are symmetrically arranged with respect to a center line along the width direction of the foot skeleton (20); and the two first deformation arms (50) located at the same side along the length direction of the foot skeleton (20) are symmetrically arranged with respect to the center line along the length direction of the foot skeleton (20), and the two first deformation arms (50) located at the same side along the width direction of the foot skeleton (20) are symmetrically arranged with respect to the center line along the width direction of the foot skeleton (20).

5. The foot skeleton (20) according to claim 3, wherein
the first groove (40) has a rectangular shape;
each of the two ends of the foot skeleton (20) along the length direction comprises one first groove (40); and
each of the two ends of the foot skeleton (20) along the length direction is separated by the first groove (40) to form two first deformation arms (50),
preferably, the foot skeleton (20) further comprises a weight reduction groove (41) at a position adjacent to the first groove (40); the weight reduction groove (41) has a rectangular shape and is in communication with the first groove (40); and a length of the weight reduction groove (41) is less than a length of the first groove (40) along the width direction of the foot skeleton (20).

6. The foot skeleton (20) according to any one of claims 3 to 5, wherein
each of the two ends of the foot skeleton (20) along the width direction comprises a notch portion (43) and two second grooves (42) in communication with the notch portion (43);
the two second grooves (42) located at the same side of the foot skeleton (20) along the width direction are spaced apart along the length direction of the foot skeleton (20); and
each of the two ends of the foot skeleton (20) along the width direction is separated by the two second grooves (42) located at the same side to form two second deformation arms (52).

7. The foot skeleton (20) according to any one of claims 1 to 6, wherein an axial direction of the deformation hole (60) extends along a width direction of the at least one deformation arm (50, 52).

8. The foot skeleton (20) according to claim 7, wherein the deformation hole (60) comprises an opening end (61), and the opening end (61) runs through a top surface or a bottom surface of the at least one deformation arm (50, 52);
wherein the deformation hole (60) comprises a first hole portion (62) and a second hole portion (63) in communication with the first hole portion (62); the second hole portion (63) comprises the opening end (61), an axial direction of the first hole portion (62) and an axial direction of the second hole portion (63) both extend along the width direction of the at least one deformation arm (50, 52); and the first hole portion (62) and the second hole portion (63) are connected by a bent portion,
preferably, a diameter of the first hole portion (62) and a diameter of the second hole portion (63) are equal or a diameter of the first hole portion (62) exceeds a diameter of the second hole portion (63); and/or
the first hole portion (62) and the second hole portion (63) are perpendicular to each other; and/or
the bent portion between the first hole portion (62) and the second hole portion (63) is an circular arc transition structure.

9. The foot skeleton (20) according to claim 7 or 8, wherein each of two ends of the deformation hole (60) along the axial direction runs through sidewalls at two sides of the at least one deformation arm (50, 52) along the width direction; or
at least one of the two ends of the deformation hole (60) along the axial direction runs through a sidewall of the at least one deformation arm (50, 52) along the width direction; or
neither of the two ends of the deformation hole (60) along the axial direction runs through sidewalls at two sides of the at least one deformation arm (50, 52) along the width direction.

10. A foot structure, comprising a force equalizing plate (10) and at least one foot skeleton (20) according to any one of claims 1 to 9, the at least one foot skeleton (20) being connected to the force equalizing plate (10).

11. The foot structure according to claim 10, wherein the foot structure further comprises a plurality of fasteners (70), the fastener (70) passes through the at least one deformation arm (50, 52) and is detachably connected to the force equalizing plate (10), portions of the foot skeleton (20) and the force equalizing plate (10) corresponding to the fastener (70) abut against each other, and a gap (11) is defined between remaining portions of the foot skeleton (20) and the force equalizing plate (10); or
the force equalizing plate (10) and the foot skeleton (20) are integrally formed, portions of the foot skeleton (20) and the force equalizing plate (10) corresponding to the force detection unit (30) abut against each other, and a gap (11) is defined between remaining portions of the foot skeleton (20) and the force equalizing plate (10).

12. The foot structure according to claim 10 or 11, wherein the force equalizing plate (10) is an integrally formed structure, the force equalizing plate (10) comprises a fore sole portion (13) corresponding to a front end of the foot skeleton (20), a rear sole portion (14) corresponding to a rear end of the foot skeleton (20), and a foot arch connected between the fore sole portion (13) and the rear sole portion (14), and the fore sole portion (13), the rear sole portion (14) and the foot arch are integrally formed; or
the force equalizing plate (10) comprises a fore sole portion (13) and a rear sole portion (14) separated from each other, the fore sole portion (13) corresponds to a front end of the foot skeleton (20) and is connected to the foot skeleton (20), and the rear sole portion (14) corresponds to a rear end of the foot skeleton (20) and is connected to the foot skeleton (20).

13. The foot structure according to any one of claims 10 to 12, wherein the foot structure further comprises a flexible pad, and the flexible pad is arranged at a side of the force equalizing plate (10) facing away from the foot skeleton (20),
preferably, the force equalizing plate (10) is integrally formed, the force equalizing plate (10) comprises a fore sole portion (13) corresponding to a front end of the foot skeleton (20), a rear sole portion (14) corresponding to a rear end of the foot skeleton (20), and a foot arch (15) connected between the fore sole portion (13) and the rear sole portion (14), the fore sole portion (13), the rear sole portion (14) and the foot arch (15) are integrally formed, and the fore sole portion (13) and the rear sole portion (14) are both provided with the flexible pad; or
the force equalizing plate (10) comprises a fore sole portion (13) and a rear sole portion (14) separated from each other, the fore sole portion (13) corresponds to a front end of the foot skeleton (20) and is connected to the foot skeleton (20), the rear sole portion (14) corresponds to a rear end of the foot skeleton (20) and is connected to the foot skeleton (20), and the fore sole portion (13) and the rear sole portion (14) are both provided with the flexible pad.

14. A robotic leg, comprising a leg structure and a foot structure according to any one of claims 10 to 13, the foot structure being connected to the leg structure.

15. A legged robot, comprising at least one robotic leg according to claim 14.
